# EUROPEAN PATENT APPLICATION

(11) **EP 1 897 582 A1**
(43) Date of publication of application: **12.03.2008**
(21) Application number: 06120335.2
(22) Date of filing: 08.09.2006
(51) Int. Cl.: A61M 25/09, A61B 5/0215, A61B 18/14, H01R 13/52

(54) **Male connector for a guide wire mounted sensor**

(71) Applicant: RADI MEDICAL SYSTEMS AB, 754 50 Uppsala (SE)
(72) Inventor: von Malmborg, Pär, 755 97, UPPSALA (SE); Gustafsson, Pär, 753 22, UPPSALA (SE)
(74) Representative: Holmberg, Nils Anders Patrik

(57) **Abstract**

A male connector (13; 20; 30; 40) for a sensor and guide wire assembly (1) for intravascular measurements of a physiological variable in a living body comprises a number of conductive members (12) separated by insulating materials (14, 15, 16; 23, 24, 25; 33, 34; 43). According to the invention the insulating material has a hydrophobic outer surface (16; 25; 33; 43) to prevent electrically conductive contaminations from forming a superficial, electrically conductive path between two neighbouring conductive members.

## Description

### Field of the Invention

The invention relates generally to sensors mounted on guide wires for intravascular measurements of physiological variables in a living body, and in particular to the design of the connector part of such sensor guide wires.

### Background of the Invention

Sensor and guide wire assemblies in which a sensor, adapted for measurements of physiological variables in a living body, such as blood pressure and temperature, is mounted at a distal portion of a guide wire are known.

For example, the U.S. Patent No. Re. 35,648, which is assigned to the present assignee, discloses a sensor and guide wire assembly comprising a sensor element, an electronic unit, signal transmitting cables connecting the sensor element to the electronic unit, a flexible tube having the signal cables and the sensor element disposed therein, a solid metal wire, and a coil attached to the distal end of the solid wire. The sensor element comprises a pressure sensitive device, e.g. a membrane, with piezoresistive elements electrically connected in a Wheatstone bridge-type of circuit arrangement mounted thereon.

The sensor guide wire ends proximally in a male connector, which is adapted for insertion into a corresponding female connector. In the U.S. Patent No. 5,938,624, which is assigned to the present assignee, an example of such a male connector is disclosed. This male connector comprises a core wire and conductive members spaced apart longitudinally along the core wire. Each conductive member is electrically connected to a signal cable, which is connected to the Wheatstone bridge circuit arranged at the sensor element in a distal portion of the sensor guide wire. The conductive members are electrically insulated from each other, and also from the core wire, by insulating material, which is disposed between the core wire and the conductive members as well as between the conductive members themselves, such that the insulating material has an outer surface which is coextensive with the outer surfaces of the conductive members.

As is discussed in the US 5,938,624, there is a potential risk of a short circuit in a male connector of the aforementioned type. A short circuit may in particular occur when the male connector is disconnected from and subsequently reconnected to the corresponding female connector, an operation that, for example, is carried out when a doctor passes and advances a balloon catheter over the sensor guide wire for treatment of a stenosis located somewhere in a patient's cardiovascular system. In such an operation there is an evident risk that the male connector is contaminated with blood, saline, water, cleaning agents, contrast media, or other electrically conductive fluids or agents. Apparently, a thin film of a conductive fluid, which superficially extends between and electrically connects two neighbouring conductive members, lowers drastically the electrical resistance between these two conductive members and creates an electric situation similar to a short circuit, which, in turn, at least temporarily can render the sensor measurements useless. Although the design suggested in the aforementioned US 5,938,624 facilitates cleaning of the male connector prior to the reinsertion into a female connector, there is still a risk that residues from the contamination of blood or other fluids lead to a short circuit, or at least unreliable performance, of the male connector.

The contamination problem illustrated above is addressed also in the international patent publication WO 99/13535, which is assigned to the present assignee. Here a solution is presented which involves a female connector provided with a wiper device for the cleaning of a male connector to be received within the female connector. Although a wiper device reduces the contamination problem, the risk of a short circuit still prevails, in particular if the male and female connectors are repeatedly disconnected and reconnected.

Consequently, there is still a need for an improved male connector which ensures proper functioning and eliminates, or at least minimizes, the risk that contaminations on the surface of the male connector cause a short circuit, leakage current, or unreliable performance.

### Summary of the Invention

The above-mentioned object is achieved by the present invention according to the independent claims.

Preferred embodiments are set forth in the dependent claims.

Embodiments of the present invention are directed to a sensor and guide wire assembly comprising a sensor element arranged in a sensor guide wire having a distal tip and comprising a core wire, a proximal tube, and at least one electrical signal transmitting cable. The sensor element is mounted at a distal portion of the core wire, and is connected to the one or more electrical signal cables, which extend from the sensor element to the proximal end portion of the sensor guide wire, where each electrical cable is connected to a respective conductive member. The conductive members are electrically insulated from the core wire and proximal tube as well as from each other by insulating material, and are arranged longitudinally spaced from each other at the proximal end portion of the sensor guide wire, so as to form a male connector for insertion into and further connection to a corresponding female connector of an external signal conditioning and display unit. Although not necessary prerequisites for the present invention, the sensor guide wire can further be fitted with a jacket as well as a distal coil, which surrounds the distal portion of the core wire and extends between the distal tip and the jacket. The sensor element is disposed inside the jacket, and is through a window in the jacket in fluid communication with the surrounding medium, e.g. blood.

According to one embodiment of the present invention, the insulating material, which electrically insulates the conductive members of the male connector from each other, is made from a hydrophobic material. According to other embodiments, a hydrophobic material is provided as a coating or layer on top of an insulating material.

By providing the insulating portions of a male connector for a sensor guide wire with a hydrophobic outer surface, contaminations, e.g. in the form of bodily fluids, saline, water, cleaning agents, contrast media, are prevented from forming an electrically conductive surface path extending between two neighbouring conductive members, to thereby eliminate or at least minimize the risk of a significant and undesired decrease in the electrical resistance between these two conductive members.

### Brief Description of the Drawings

Fig 1 is a schematic illustration of a sensor and guide wire assembly comprising a male connector according to a first embodiment of the present invention, wherein the male connector comprises a hydrophobic material arranged as an outer layer on top of an electrically insulating material.
Fig. 2 is a cross-sectional view of the male connector shown in Fig. 1.
Fig. 3 is a cross-sectional view of a second embodiment of a male connector for a sensor and guide wire assembly, wherein the male connector comprises a hydrophobic material provided as a thin coating on an electrically insulating material.
Fig. 4 is a cross-sectional view of a third embodiment of a male connector for a sensor and guide wire assembly, wherein the male connector comprises an electric insulation in the form of a hydrophobic layer.
Fig. 5 is a cross-sectional view of a fourth embodiment of a male connector for a sensor and guide wire assembly, wherein the male connector comprises an electric insulation in the form of a homogeneous, hydrophobic insulating material.
Fig. 6 illustrates the concept of contact angle, which is used to define the degree of hydrophobicity possessed by the hydrophobic materials described herein.

### Detailed Description of Preferred Embodiments

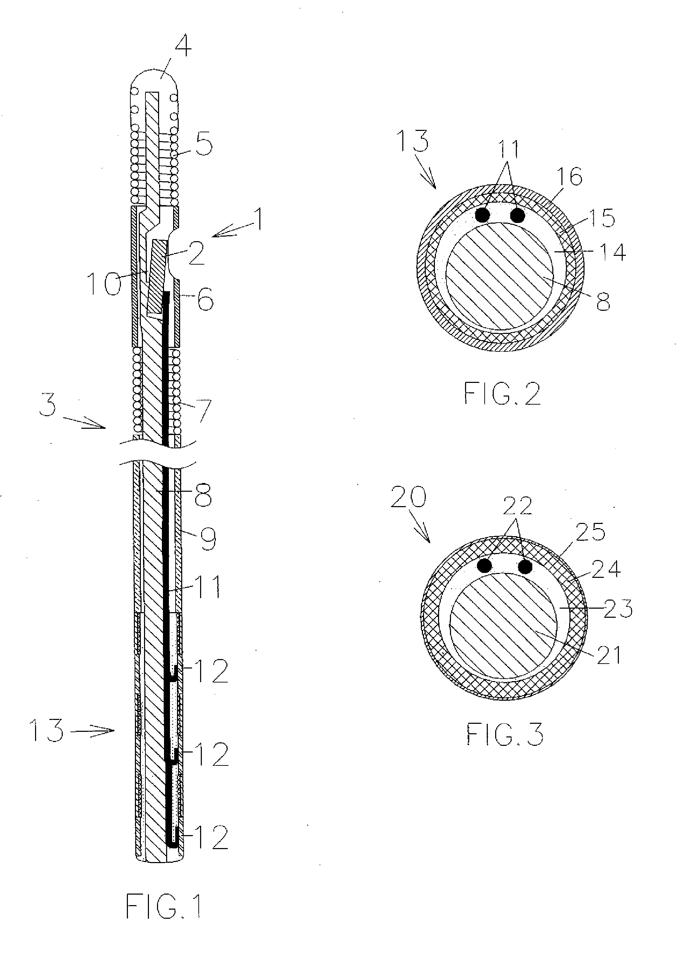

Fig. 1 illustrates schematically the general design of a sensor and guide wire assembly 1 according to a first embodiment of the present invention. The sensor and guide wire assembly 1 comprises a sensor element 2, which is arranged in a distal portion of a sensor guide wire 3. More specifically, the sensor guide wire 3 comprises a distal tip 4, a distal coil spring 5, a jacket or sleeve 6, a proximal coil spring 7, a core wire 8, and a proximal tube 9. The distal coil spring 5 is attached to the distal tip 4 and extends to the jacket 6, which serves as a housing for the sensor element 2. The proximal coil spring 7 extends between the jacket 6 and the proximal tube 9. The sensor element 2 is mounted in a recess 10 in a distal portion of the core wire 8, and is through a window in the jacket 6 in fluid communication with the medium, e.g. blood, surrounding the sensor and guide wire assembly 1. The sensor and guide wire assembly 1 comprises further a number of signal transmitting cables 11, the distal ends of which are electrically connected to the sensor element 2 and which extend along the core wire 8 to the proximal end portion of the sensor and guide wire assembly 1, where each signal transmitting cable 11 is electrically connected to a conductive member 12. The conductive members 12 are electrically insulated from each other as well as from the core wire 8 by insulating materials, so as to form a male connector 13 adapted for connection to a corresponding female connector of an external signal conditioning and display unit (not shown in Fig. 1).

As is best seen in Fig. 2, where a cross-section of the male connector 13 is illustrated, the insulating materials comprise three layers: an inner layer 14, a middle layer 15, and an outer layer 16. The middle layer 15 can, as is known in the prior art, be made from an insulating material such as polyimide, and can be provided in the form of a number of insulating tube members 15, which are separating the conductive members 12 from each other. The most distal insulating member 15 separates the most distal conductive member 12 from the proximal tube 9. The inner layer 14 is typically made from an insulating material, such as epoxy, which in its liquid form can be provided by means of the capillary effect arising between the core wire 8 and the conductive members 12 as well as between the core wire 8 and the insulating members 15, to thereby completely fill the space within the male connector 13. In accordance with the present invention, the outer layer 16 is made from a hydrophobic material, which can be applied by painting, dipping, spraying, or other techniques. Examples of highly hydrophobic materials are silicone, Teflon®, and FEP (Fluorinated Ethylene Propylene). In a male connector having an outer diameter of 350 µm (0.014 inches), the aggregate thickness of the insulating middle layer 15 and the outer hydrophobic layer 16 may be about 50 µm, with the thickness of the hydrophobic layer 16 (or the thickness of the insulating layer 15) ranging from a few microns to almost the maximal available thickness of about 50 µm, where the upper limit depends on the desired diameter of the core wire 8. In other words, there is a large degree of freedom in designing a hydrophobic male connector, the important consideration being that the male connector, between the conductive members, is provided with a hydrophobic outer surface, which, in an extreme case, could be only a few Angstroms thick.

The latter statement implies that a hydrophobic material can be applied as a thin coating on the surface of an insulating material. This feature is illustrated in Fig. 3, where a second embodiment of a male connector 20 is shown in cross-section. The male connector 20 comprises a core wire 21, a number of signal cables 22, an inner insulating layer 23, a middle insulating layer 24, and an outer hydrophobic coating 25. As in the first embodiment shown in Figs. 1 and 2, the inner insulating layer 23 can consist of epoxy, which during the manufacture can be provided in its liquid form by utilizing the capillary effect. The middle insulating layer 24 can be applied in the form of solid rings, liquid, flexible material, or other form, and can comprise polyimide, whereas the coating 25 comprises a highly hydrophobic material, such as silicone, PTFE (polytetrafluoroethylene), or a lipid.

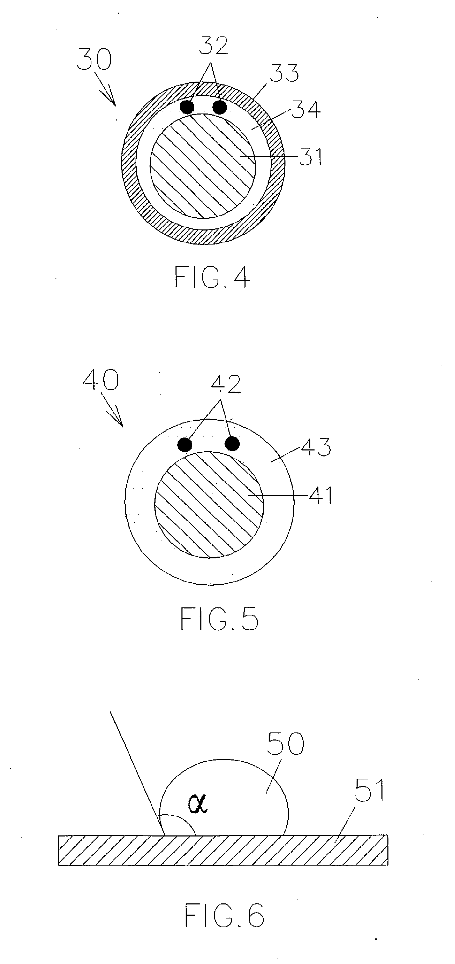

In the embodiment shown in Fig. 3, and in particular in the embodiment illustrated in Figs. 1 and 2, it is possible to omit the middle insulating layer, i.e. to only arrange a hydrophobic layer, which provides the male connector with both the insulating as well as hydrophobic properties. An exemplifying embodiment of this variant is disclosed in Fig. 4, where a male connector 30 comprises a core wire 31, a number of signal cables 32, and a hydrophobic insulating layer 33. The space between the core wire 31 and the hydrophobic insulating layer 33 is in this example filled with a suitable insulating material 34, such as epoxy or silicone. It is, however, possible to omit such filling, in particular if each of the signal cables is provided with its own electrically insulating coating. Also a core wire can be provided with an electrically insulating coating or layer. In a male connector having an outer diameter of 350 µm (0.014 inches), the thickness of the hydrophobic insulating layer 33 can range from a few Angstroms to about 50 µm. The upper limit may depend, however, on the diameter required for the core wire 31.

Instead of providing a hydrophobic material as a layer or coating, and in some applications in the form of separate pieces of such a hydrophobic material, it is also possible to provide a male connecter in which the core wire and signal cables are embedded in a hydrophobic material. Fig. 5 discloses such an embodiment of the present invention. Here a male connector 40 comprises a core wire 41, a number of signal cables 42, and a homogeneous, hydrophobic insulating material 43. The insulating material 43 can comprise or consist of silicone; and the male connector 40 can be manufactured in one piece, e.g. by moulding.

As is generally accepted, the hydrophobicity of a material is expressed in terms of contact angle. This concept is illustrated in Fig. 6, where a drop 50 of a fluid has formed on a substrate 51. The contact angle α is defined as the angle between the plane of the substrate 51 and the tangent to the boundary or wall of the fluid drop 50. Small contact angles imply that the drop has a tendency to spread out on the substrate, whereas large contact angles imply that the drop holds together. Theoretically, the contact angle depends on the substrate as well as the fluid, but water is the common test fluid. Materials having contact angles larger than 90° are characterized as highly hydrophobic materials. Water on Teflon® has, for example, a contact angle of about 112°, which is very high.

The hydrophobic materials used in a male connector according to the present invention preferably have contact angles larger than 90°, and more preferably larger than 100°, and most preferably larger than 110°. Examples of suitable hydrophobic materials are: silicone, PTFE (Teflon®), FEP, and lipids. The contact angle for a certain hydrophobic material may be enhanced by surface treatment, e.g. by providing a roughened surface.

As described above, a male connector comprises a number of conductive members. These members, which typically are made from a metal, exhibit relatively small contact angles, and have thereby a low hydrophobicity. If a fluid, such as blood, water, saline, cleaning agent, or contrast medium, is deposited on the surface of a male connector provided with conductive members, which have a low hydrophobicity, and insulating members, which have a high hydrophobicity, in an alternating structure, the fluid has consequently a tendency to accumulate at the surface of the conductive members, something which - in accordance with the discussion above - is an advantageous effect, because no continuous, electrically conductive surface paths are formed between the conductive members. This positive effect can be enhanced by further lowering the hydrophobicity of the conductive members. This can be accomplished by suitable surface treatment, e.g. by providing the conductive members with a roughened surface. It is preferred that the conductive members have an outer surface that is less hydrophobic than the outer surface of the insulating material arranged between the conductive members.

Although the present invention has been described with reference to specific embodiments, also shown in the appended drawings, it will be apparent for those skilled in the art that many variations and modifications can be done within the scope of the invention as described in the specification and defined with reference to the claims below. It should in particular be noted that the sensor and guide wire illustrated herein is merely an example of such a device, and that many variations are known and could be done, especially regarding parts located distally of a proximal male connector. For example, the core wire in a male connector may be the same core wire that extends in the sensor guide, or can be a core wire that is separate from the core wire in the sensor guide. It should also be noted that in all embodiments disclosed above, the core wire and/or the signal cables can be provided with their own electrical insulation, e.g. in the form of a coating. In other words, a male connector according to the present invention can be provided for almost any type of guide wire mounted sensor. It should further be noted that the hydrophobic material can be provided as a continuous material, e.g. by moulding, or in the form of separate, preferably tubular members, the important feature being that the material presents a hydrophobic outer surface.

## Claims

1. Male connector for a guide wire mounted sensor for intravascular measurements of a physiological variable in a living body, said male connector comprising a number of conductive members separated by insulting material, **characterized in that** insulating material has a hydrophobic outer surface.

2. Male connector according to claim 1, **characterized in that** the hydrophobic outer surface exhibits a contact angle larger than 90°.

3. Male connector according to claim 1, **characterized in that** the hydrophobic outer surface exhibits a contact angle larger than 100°.

4. Male connector according to claim 1, **characterized in that** the hydrophobic outer surface exhibits a contact angle larger than 110°.

5. Male connector according to claim 1, wherein the conductive members have an outer surface that is less hydrophobic than an outer surface of the insulating material.

6. Male connector according to any preceding claim, wherein the insulating material is made from silicone.

7. Male connector according to any of claims 1-5, wherein the insulating material is made from polytetrafluoroethylene (PTFE).

8. Sensor guide wire with a male connector comprising a number of conductive members separated by insulating material, **characterized in that** the insulating material has a hydrophobic outer surface.

9. Sensor guide wire with a male connector according to claim 8, wherein the hydrophobic outer surface exhibits a contact angle larger than 90°.

10. Sensor guide wire with a male connector according to claim 8, wherein the conductive members have an outer surface that is less hydrophobic than an outer surface of the insulating material.

## Amended claims

### Amended claims in accordance with Rule 137(2) EPC.

**1.** Male connector for a guide wire mounted sensor for intravascular measurements of a physiological variable in a living body, said male connector comprising a number of conductive members arranged longitudinally spaced from each other and separated by insulating material, **characterized in that** insulating material has a hydrophobic outer surface.

**2.** Male connector according to claim 1, wherein the conductive members have an outer surface that is less hydrophobic than an outer surface of the insulating material.

**3.** Male connector according to any preceding claim, wherein the insulating material is made from silicone.

**4.** Male connector according to any of claims 1-3, wherein the insulating material is made from polytetrafluoroethylene (PTFE).

**5.** Male connector according to any of claims 1-4, wherein the insulating material is made from Flourinated Ethylene Propylene (FEP).

**6.** Male connector according to any of claims 1-5, wherein the insulating material is made from lipids.

**7.** Sensor guide wire with a male connector comprising a number of conductive members arranged longitudinally spaced from each other and separated by insulating material, **characterized in that** the insulating material has a hydrophobic outer surface.

**8.** Sensor guide wire with a male connector according to claim 7, wherein the conductive members have an outer surface that is less hydrophobic than an outer surface of the insulating material.
